# EUROPEAN PATENT SPECIFICATION

(11) **EP 3 559 675 B1**
(45) Date of publication and mention of the grant of the patent: **16.08.2023**
(21) Application number: 17840505.6
(22) Date of filing: 21.12.2017
(51) Int. Cl.: G01N 33/68, G01N 30/02, G01N 30/88

(54) **A METHOD FOR MONITORING OF AMINO ACIDS IN BIOLOGICAL MATERIAL**
VERFAHREN ZUR ÜBERWACHUNG VON AMINOSÄUREN IN BIOLOGISCHEM MATERIAL
PROCÉDÉ PERMETTANT DE SURVEILLER DES ACIDES AMINÉS DANS UN MATÉRIAU BIOLOGIQUE

(30) Priority: 22.12.2016 PL 41996016; 16.10.2017 PL 42317617
(43) Date of publication of application: 30.10.2019
(73) Proprietor: Gdanski Uniwersytet Medyczny, 80-210 Gdansk (PL)
(72) Inventor: KONIECZNA, Lucyna, 84-240 Reda (PL); BACZEK, Tomasz, 80-174 Gdansk (PL); NIEDZWIECKI, Maciej, 80-506 Gdansk (PL); PYSZKA, Magdalena, 80-366 Gdansk (PL); OKONSKA, Magdalena, 80-136 Gdansk (PL)
(74) Representative: Rzazewska, Dorota
(86) International application number: PCT/PL2017/000130
(87) International publication number: WO 2018/117881

(56) References cited:
- PRINSEN HUBERTUS C ET AL: "Rapid quantification of underivatized amino acids in plasma by hydrophilic interaction liquid chromatography (HILIC) coupled with tandem mass-spectrometry", JOURNAL OF INHERITED METABOLIC DISEASE, KLUWER, DORDRECHT, NL, vol. 39, no. 5, 21 April 2016 (2016-04-21) , pages 651-660, XP036033275, ISSN: 0141-8955, DOI: 10.1007/S10545-016-9935-Z [retrieved on 2016-04-21]
- LUNING SUN ET AL: "Hydrophilic interaction liquid chromatography coupled with tandem mass spectrometry method for the simultaneous determination of l-valine, l-leucine, l-isoleucine, l-phenylalanine, and l-tyrosine in human serum", JOURNAL OF SEPARATION SCIENCE., vol. 38, no. 22, 1 November 2015 (2015-11-01), pages 3876-3883, XP055458157, DE ISSN: 1615-9306, DOI: 10.1002/jssc.201500512
- GIUSEPPE PAGLIA ET AL: "Monitoring metabolites consumption and secretion in cultured cells using ultra-performance liquid chromatography quadrupole-time of flight mass spectrometry (UPLC-Q-ToF-MS)", ANALYTICAL AND BIOANALYTICAL CHEMISTRY, SPRINGER, BERLIN, DE, vol. 402, no. 3, 13 December 2011 (2011-12-13), pages 1183-1198, XP019993316, ISSN: 1618-2650, DOI: 10.1007/S00216-011-5556-4
- CONVENTZ ET AL: "Simultaneous determination of 3-nitrotyrosine, tyrosine, hydroxyproline and proline in exhaled breath condensate by hydrophilic interaction liquid chromatography/electrospray ionization tandem mass spectrometry", JOURNAL OF CHROMATOGRAPHY B: BIOMEDICAL SCIENCES & APPLICATIONS, ELSEVIER, AMSTERDAM, NL, vol. 860, no. 1, 19 November 2007 (2007-11-19), pages 78-85, XP022364475, ISSN: 1570-0232, DOI: 10.1016/J.JCHROMB.2007.10.031
- KONIECZNA LUCYNA ET AL: "Hydrophilic interaction chromatography combined with dispersive liquid-liquid microextraction as a preconcentration tool for the simultaneous determination of the panel of underivatized neurotransmitters in human urine samples", JOURNAL OF CHROMATOGRAPHY A, ELSEVIER, AMSTERDAM, NL, vol. 1431, 23 December 2015 (2015-12-23), pages 111-121, XP029388326, ISSN: 0021-9673, DOI: 10.1016/J.CHROMA.2015.12.062
- LUCYNA KONIECZNA ET AL: "Bioanalysis of underivatized amino acids in non-invasive exhaled breath condensate samples using liquid chromatography coupled with tandem mass spectrometry", JOURNAL OF CHROMATOGRAPHY A, vol. 10, 1 February 2018 (2018-02-01), XP055458026, AMSTERDAM, NL ISSN: 0021-9673, DOI: 10.1016/j.chroma.2018.02.019

## Description

The subject of the invention is a new method for monitoring of the level of amino acids in biological material in which the following stages of the analysis of amino acids level in the material may be distinguished: preparation of the biological sample for the chromatographic analysis, separation by liquid chromatography coupled with tandem mass spectrometry, separation of 21 amino acids is carried out through hydrophilic interaction chromatography (HILIC) with the use of columns packed with polar sorbents. Application of the abovementioned method in order to determine the level of amino acids in biological material obtained from subjects with selected type of leukaemia and/or with type 1 diabetes is also the topic of the invention.

The term leukaemia refers to cancer of the haematopoietic system. Correct haematopoiesis is disturbed through a genetic mutation which stops the production of mature and functional cells of the circulatory system in the described disease. Non-differentiated cells infiltrate bone marrow and other tissues of the organism and begin to appear in peripheral blood.

Classification of leukaemia in children is based on a dominant cell line, and on a degree of differentiation of these cells. Consequently, the terms myeloid leukaemia and lymphoblastic leukaemia refers to the cell types involved in the development and progression of particular cancers.

Cancer is one of the more frequent causes of death among children. It is estimated that around 1200 people in Poland develop this disease each year. Approximately 20% of all cancers occurring in children are leukaemias. They usually occur between the age of 2 and 7, more often in males. Cancerous lesions affect white blood cells. The ethiology of the disease has not yet been fully explored. It is assumed that the appearance of cancer is a result of several factors. The genetic background is considered to be the crucial one. Congenital chromosome disorders such as Down syndrome, Klinefelter syndrome, Fanconi syndrome, Shwachman-Diamond syndrome, Bloom syndrome, and Nijmegen breakage syndrome increase the risk of leukaemia occurrence, e.g. Down syndrome increases the risk by 10-20%. Environmental factors, especially exposure to benzene, chlorine derivatives of hydrocarbons, and ionising radiation, may cause the activation of a cascade of oncogenes and thus contribute to the process of carcinogenesis. Several types and subtypes of leukaemia can be differentiated. As far as the course of disease is concerned, two basic groups can be distinguished: acute and chronic leukaemias. If a location of changes is considered as the criterion - leukaemias can be divided into myeloid and lymphoblasticones.

Acute lymphoblastic leukaemia (ALL) which constitutes 80% of all leukaemias is the most frequent leukaemia occurring in children.

Treatment of acute lymphoblastic leukaemia is based on chemotherapy - the following stages can be differentiated: remission induction, remission consolidation, and maintenance treatment. In the initial phase of treatment vincristine and daunorubicin are applied once a week. Additionally, L-asparaginase, prednisolone, cyclophosphamide, and cytosine arabinoside are also administered. Methotrexate is delivered intrathecally for prevention of the CNS (central nervous system). In the subsequent phase of treatment cyclophosphamide, etoposide, and methotrexate are used. During the maintenance treatment a patient is given methotrexate, and 6-mercaptopurine. In addition to that, every few weeks vincristine, anthracycline, and prednisolone are given for re-induction purpose. Properly selected chemotherapy constitutes a 70% chance of recovery.

In children, the acute non-lymphoblastic leukaemia such as acute myeloid leukaemia (AML) is diagnosed less frequently, - constituting only 15% of all acute leukaemias. This type of cancer is more often characterised with extramedullary structures infiltration, such as the central nervous system, and bones.

Treatment of acute myeloid leukaemia is also composed of several stages, and ends with success in 43% of cases of disease. At first, daunorubicin, and cytarabine are used often in combination with idarubicin. Methotrexate is delivered intrathecally for prevention of the CNS. In the subsequent stages of treatment cytosine arabinoside, methotrexate, daunorubicin, and tioguanine are applied. Additionally, transplantation of haematopoietic cells (which gives 45-60% chance of recovery) is applied.

Only chronic myeloid leukaemia occurs in children. It constitutes less than 5% of leukaemias diagnosed in this age group. Its diagnosis is based on blood testing in which hyperleukocytosis, and anaemia are confirmed.

Changes in the composition of cerebrospinal fluid may help to diagnose some diseases.

Examination of changes in the level of amino acids in cerebrospinal fluid obtained from children with acute lymphoblastic leukaemia is significant as it may allow identification of potential response to treatment markers. The results of research shows that the level of glutamine in cerebrospinal fluid of children suffering from cancer was significantly higher at the moment of diagnosis than in the control group. A decreasing level of this amino acid was also observed during treatment.

The basis of leukaemia classification is the assumption that leukaemia cells are the population of cells which has stopped at a certain stage of maturation and differentiation. Based on the origin of "damaged" cells from the haematopoietic type of cell, acute lekaemias are divided into lymphoblastic (ALL), and myeloid (AML). Acute lymphoblastic leukaemia is a heterogeneous disease. At this stage, according to FAB classification, taking into consideration morphotic criteria dependent on the size of a cell, the shape of a nucleus, and the content of the cytoplasm, the following forms can be differentiated: L1, L2, L3. Contemporary diagnostics of acute leukaemias comprises not only morphological evaluation of marrow's and peripheral blood's blast cells but also cytochemical and cytogenetic examination, and indication of the immunophenotype of these cells. Phenotypic classification is based on known stages of maturation and phenotypic differentiation of the cells of lymphocyte B or T. In our project we examined children diagnosed with acute leukaemia derived from line B lymphocyte.

Prinsen Hubertus C et al., "Rapid quantification of underivatized amino acids in plasma by hydrophilic interaction liquid chromatography (HILIC) coupled with tandem mass-spectrometry", Journal of Inherited Metabolic Disease, Kluwer, Dordrecht, NL, vol. 39, no. 5, refers to a chromatographic method for amino acid determination in plasma samples, but not in cerebrospinal fluid. Our proposed method was used for the analysis of unique biological samples - cerebrospinal fluid obtained from leukemia children. Chromatographic conditions introduced are different than in our work. In this publication authors did not present results of AA level in CSF samples but in plasma. Chromatographic conditions introduced and the sample preparation procedure are different than in our work. The volume of plasma was different, 40 µl in D1 versus 50 µl in our method. The authors of the publication diluted the sample seven times with buffer, whereas we used the sample to be concentrated by evaporation to dryness and reconstituted before LC-MS injection in order to finally obtain a lower limit of detection and quantification. This optimization of LC-MS parameters allowed to qualitative and the quantitative determination of 21 amino acids in our study while in publication presented separation of 36 AA, unfortunately, including 12 only qualitatively, not quantitatively. The scientific journal is not a typical analytical and chromatographic journal, we have doubts about the validation of the described method. Accuracy and precision should be calculated for each point of calibration range, not for one concentration level. From the Inventor's experience, it is unlikely that the column will allow 1500 analyzes, it is unbelievably much, especially for HILIC column.

Luning Sun et al, "Hydrophilic interaction liquid chromatography coupled with tandem mass spectrometry method for the simultaneous determination of 1-valine, 1-leucine, 1-isoleucine, 1-phenylalanine, and 1-tyrosine in human serum", Journal of Separation Science, DE, vol. 38, no. 22, discloses the LC-MS method developed for simultaneous determination only 5 AA in human serum obtained from patients with diabetes type 2, no for cancer patients with leukemia and no for diabetes type 1. Chromatographic separation was carried out on a Syncronis HILIC column 150 x 2.1 mm with 5 µm of a particle, we proposed more polar column- Amide XBridge. In publication applied completely another mobile phase with high hyper-saline 120 mM ammonium acetate and a temperature of 35 degrees. In general experience, 120 mM ammonium acetate buffer is too concentrated, hence such buffers make it difficult to ionize and mass detector response would decrease and could cause ion suppression. Authors represent differ sample preparation procedure based on dilution with water and extraction with acetonitrile. Finally, our procedure including the evaporation step significantly improved the limit of detection and limit of quantification of the method.

Giuseppe Paglia et al., "Monitoring metabolites consumption and secretion in cultured cells using ultra-performance liquid chromatography quadrupole-time of flight mass spectrometry (UPLC-Q-ToF-MS)", Analytical and Bioanalytical Chemistry, Springer, Berlin, DE, (20111213), vol. 402, no. 3, concerns an untargeted and targeted analysis of metabolites including amino acids. The Acquity amide column with other dimensions 2.1 × 150 mm, 1.7 µm was applied. Another phase composition based on 100% acetonitrile (A) and water (B), both containing 0.1% formic acid, compared to our proposed method was used.

The sample preparation procedure was processed by adding 200 µl of acetonitrile to 50 µl of culture medium samples without evaporation step versus our procedure containing the evaporation step. It makes our method more useful for clinical samples because of low volume of biological material.

Conventz et al, "Simultaneous determination of 3-nitrotyrosine, tyrosine, hydroxyproline and proline in exhaled breath condensate by hydrophilic interaction liquid chromatography/electrospray ionization tandem mass spectrometry", Journal of Chromatography B Biomedical Sciences & Applications, Elsevier Amsterdam, NL, (20071119), vol. 860, refers methodology for the analysis of two amino acids L-proline and L-tyrosine and their two metabolites, trans-L-4-hydroxyproline and nitrotyrosine in exhaled breath condensate (ECB) samples We proposed simultaneous determination of 23 amino acids in EBC samples in contrast to publication. Analysis of EBC samples requires an extremely sensitive method because amino acid concentrations in these samples are highly diluted in EBC. The Authors applied the method for the evaluation of four compound levels in EBC samples obtained from healthy persons of the general population. The aim of our work was to develop and validate an analytical method for the accurate and specific simultaneous determination of a panel of compounds in EBS samples obtained from children with diagnosed leukemia. There is no mention in the literature concerning the determination of the level of amino acids in EBC samples collected from children with leukemia. Other column, such as ZIC-HILIC and mobile phase composition were performed by D4 authors (5 Mm ammonium acetate pH4).

In the publication by Lucyna Konieczna et al.: "Hydrophilic interaction chromatography combined with dispersive liquid-liquid microextraction as a preconcentration tool for the simultaneous determination of the panel of underivatized neurotransmitters in human urine samples" J. Chromatogr. A, 1431 (2016) 111-112, and Konieczna et al.: "Analytical approach to determining human biogenic amines and their metabolites using eVol microextraction in packed syringe coupled to liquid chromatography mass spectrometry method with hydrophilic interaction chromatography column", Talanta 150 (2016) 331-9 (http://doi.org/10.1016/j.talanta.2015.12.056) the analysis of neurotransmitters and their metabolites in human blood plasma, and urine has been revealed in which a method based on clean standards of above mentioned analytes was developed. However, those are different compounds in relation to the compounds that are the subject of analysis in this invention.

In this invention other precursor ions characteristic for determined amino acids are monitored based on the mass spectrum received from the analysis of reference substances by LC-MS method in positive ions mode.

Application of a connected techniques - liquid chromatography with mass spectrometry - allows an additional identification of analytes not only by the retention time but also by the mass spectrum where chosen ions (in this case precursor ions [M+H] +) are monitored. Another gradient program, time of gradient, total analysis time, and, most importantly, other ions were monitored during the chromatographic process, and with different values of fragmentation tension.

In case of neurotransmitters and their metabolites, preparation of the sample for the chromatographic analysis involves optimization, and application of microextraction techniques: dispersive liquid-liquid microextraction, and solid-phase extraction packed in the eVol syringe's needle. Microextraction techniques described in detail in the abovementioned publications have allowed for a successful isolation of these compounds from the biological matrix.

In the present invention, the preparation of the sample is completely different much simpler and cheaper than is solutions known in the prior art. It consist of deproteinization of cerebrospinal fluid by using 0.1 M of HCl, centrifugation, and evaporation of the layer from the precipitate in vacuum conditions. For the analysis of the blood plasma, 0.1 M of HCl was used with the purpose of deproteinization. Next, it was extracted by liquid-liquid method with methanol, which was then evaporated under vacuum conditions.

The purpose of this invention is to provide a new methods that can be used to detect acute lymphoblastic leukaemia and/or type 1 diabetes through determination of amino acids level.

The subject of this invention is a method for monitoring of amino acids in a biological material, wherein the amino acid analysis in the material occurs in the following steps:
a) preparation of a sample of biological material which is an exhaled breath condensate, wherein 1 mL of an internal standard is added to the sample, the internal standard is norvaline and homoarginine, labelled deuterated leucine Leu-d3,
b) centrifugation of a sample at 1000 rpm / 8 minutes,
c) subsequent evaporation to dryness at 45-60°C during 1h,
d) dissolving of a dry residue in 100 µL of a mixture of acetonitrile and H₂O in a ratio of 8:2 (v/v) and subsequent centrifugation at 10000 rpm /8 minutes, and
e) chromatographic analysis with a mass detector by liquid chromatography coupled with tandem mass spectrometry (LC-MS/MS) performed under conditions wherein:
   - 21 amino acids are separated by hydrophilic interaction chromatography (HILIC), with the use of column packed with polar sorbent,
   - a mobile phase consists of phase A): 10 mM ammonium formate (pH 3) in water and B): 10 mM ammonium formate in acetonitrile under gradient elution conditions,
   - the mobile phase flow is 0.8 - 1 mL/min,
   - the column temperature is 25°C,
   - a drying gas temperature: 250-350°C, flow of the drying gas: 8 - 12 L/min, the drying gas temperature is: 350°C, flow of the drying gas: 11 L/min, collision energy depending on the monitored production (range from 5 to 70),
   - the duration of a single analysis of 21 amino acids with column equilibration is 25 - 30 min, and amino acid separation occurred under gradient elution conditions.

The subject of this invention is also a method for monitoring of amino acids in a biological material, characterized in that comprising following steps of:
a) preparation of a sample of one biological material which is a cerebrospinal fluid or a blood plasma
b) addition of a deproteinizing agent which is a 0.1 M solution of hydrochloric acid in water for cerebrospinal fluid or 0.1 M hydrochloric acid in methanol for blood plasma,
b) centrifugation of a sample at 1000 rpm / 8 minutes,
c) subsequent evaporation to dryness at 45-60°C during 1h,
d) dissolving of a dry residue in 100 µL of a mixture of acetonitrile and H₂O in a ratio of 8:2 (v/v) and subsequent centrifugation at 10000 rpm /8 minutes, and
e) chromatographic analysis with a mass detector by liquid chromatography coupled with tandem mass spectrometry (LC-MS/MS) performed under conditions wherein:
   - 21 amino acids are separated by hydrophilic interaction chromatography (HILIC), with the use of column packed with polar sorbent,
   - a mobile phase consists of phase A): 10 mM ammonium formate (pH 3) in water and B): 10 mM ammonium formate in acetonitrile under gradient elution conditions,
   - the mobile phase flow is 0.8 - 1 mL/min,
   - the column temperature is 25°C,
   - a drying gas temperature: 250-350°C, flow of the drying gas: 8 - 12 L/min, the drying gas temperature is: 350°C, flow of the drying gas: 11 L/min, collision energy depending on the monitored production (range from 5 to 70),
   - the duration of a single analysis of 21 amino acids with column equilibration is 25 - 30 min, and amino acid separation occurred under gradient elution conditions.

The method as defined above is used for monitoring the level of amino acids in patients with lymphoblastic leukaemia during chemotherapy and/or type 1 diabetes at the time of diagnosis.

An advantage of this invention is the easily obtainable biological material, exhausts in particular, and a very simple way of carrying out the analysis.

In case, wherein the sample of biological material is an exhaled breath condensate (EBC), the method is particularly useful for patients from paediatric population.

Biological material such as blood or urine is applied with success in medical diagnosis. However, in practice the exhaled breath condensate (EBC) has been the least used biological material to date. This is probably because it is the least explored one.

Exhaled breath condensates are extremely valuable biological material, which are collected in an entirely non-invasive and safe manner that is not stressful to patient in any way, because they are collected during standard breathing.

The exhaled breath condensate is relatively free of proteins, therefore it does not require to undergo the deproteinization stage, which is an essential procedure with blood plasma, and cerebrospinal fluid. For this reason, preparation of the EBC sample is based on evaporation of the sample under vacuum, that is on its "concentration", which makes this method more economical as it saves analyst's time.

Moreover, the research performed by the creators indicated that the results of the analysis of the influence of EBC (sample) solution's pH used for administrating analytes into chromatographic system suggest the possibility of their analysis without prior pH change (which is necessary with urine samples collected non-invasively).

It is not only the patients that benefit from this non-invasive (thus, stressless) method of sample colleting, but also analysts and diagnosticians gain as the samples they examine do not require special and time-consuming preparation for their further chromatographic analysis.

The proposed new technique for collecting exhaled breath condensate allows for an unprecedented combination in the diagnostics of leukaemia and type 1 diabetes of organ specificity with total non-invasiveness. First of all, it offers a number of possibilities in the form of multiple, frequent repetition of the test, performing it in the most critical period of time from the clinical point of view (*point-of-care real-time analysis*)*,* in patients in severe conditions, and what is particularly important to us - in children. During conducting of the measurements, a patient breathes normally and calmly, the measurements do not cause any damage to airway mucosa. The collection of condensates is possible not only among children from the age of three, but even among infants, through the use of special face masks. In addition, as a non-invasive procedure, it can be repeated multiple times, giving the possibility of obtaining disease markers in real time.

The solutions provided by the invention enables detection of new risk factors in cancer diseases and type 1 diabetes in children, as well as allowing for the development of new therapeutic strategies, which would be more precisely adapted to the stage or aggressiveness of the disease. Other positive aspects such as early detection of complications of the applied treatment and the disease itself, and immediate inclusion of appropriate treatment may arise from use of method provided in invention.

The terms used above, and in the description and patent claims, have following meaning:
**Amino acids** (AA) - stands for 21 amino acids: Alanine (Ala), Arginine (Arg), Asparagine (Asn), Aspartic Acid (Asp), Cysteine (Cys), Glycine (Gly), Glutamine (Gln), Glutamine Acid (Glu), Histidine (His), Hydroxyproline (Hpro), Isoleucine (Ile), Leucine (Leu), Lysine (Lys), Methionine (Met), Phenylalanine (Phe), Proline (Pro), Serine (Ser), Threonine (Thr), Tryptophan (Trp), Tyrosine (Tyr), Valine (Val).
**Exhaled breath condensate (EBC)** - stands for the sample of exhaled breath, which is then concentrated through its evaporation under vacuum accordingly to this invention.

### Description of figures and tables:

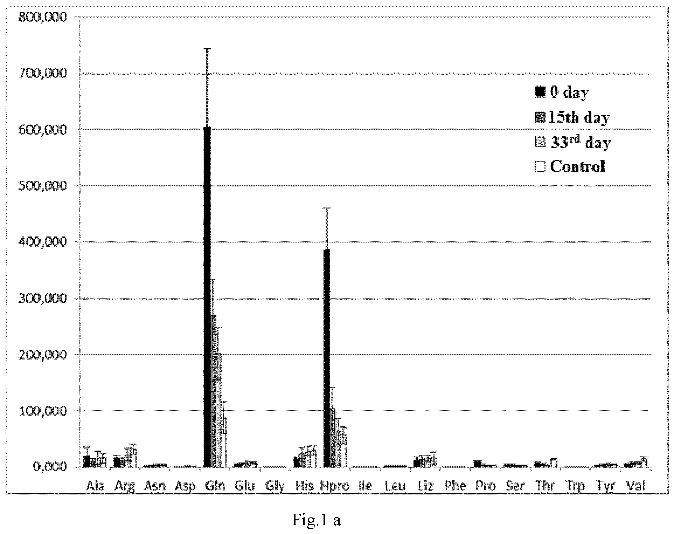

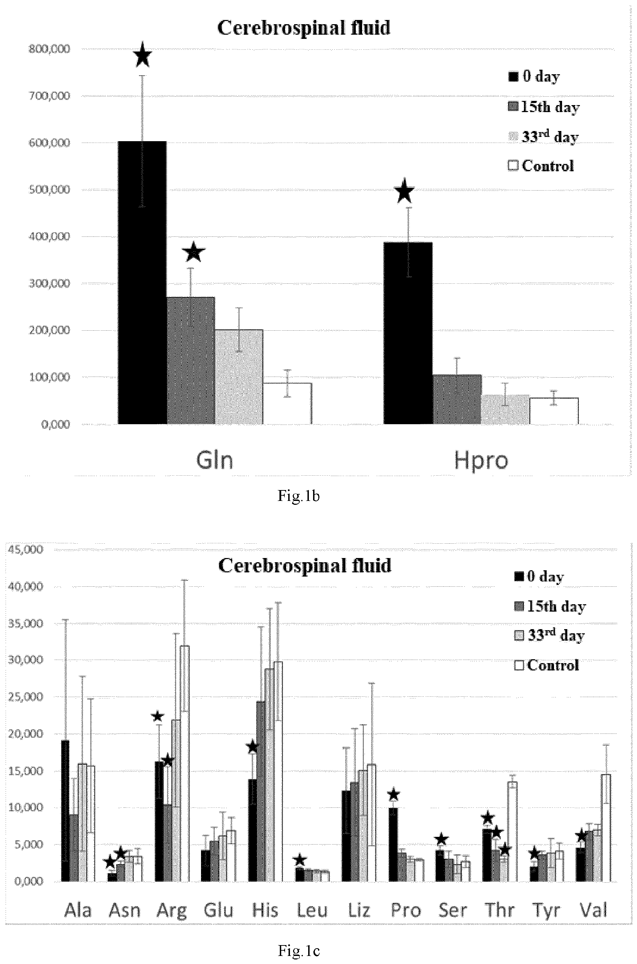

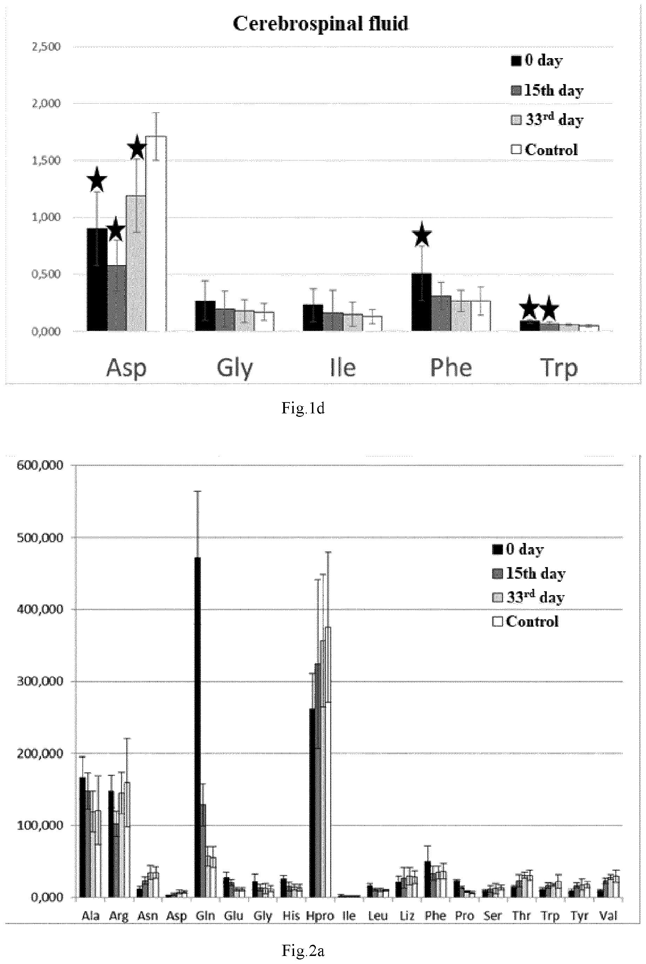

**Fig. 1** - presents average values of amino acids' concentration in the cerebrospinal fluid determined using LC-MS method; n = 40 subjects (children) a) all of the measured amino acids, b) Gln, and Hpro c) amino acids: Ala, Asn, Arg, Glu, His, Leu, Liz, Pro, Ser, Thr, Tyr, Val, excluding Gln, and Hpro d) remaining amino acids excluding the ones mentioned in point b) and c). Standard deviation is visually represented in Figs a-d, and statistically significant differences between average concentration of amino acid of interest obtained from ALL leukaemia group and from the control group are marked with * and represented in Fig. b-d.

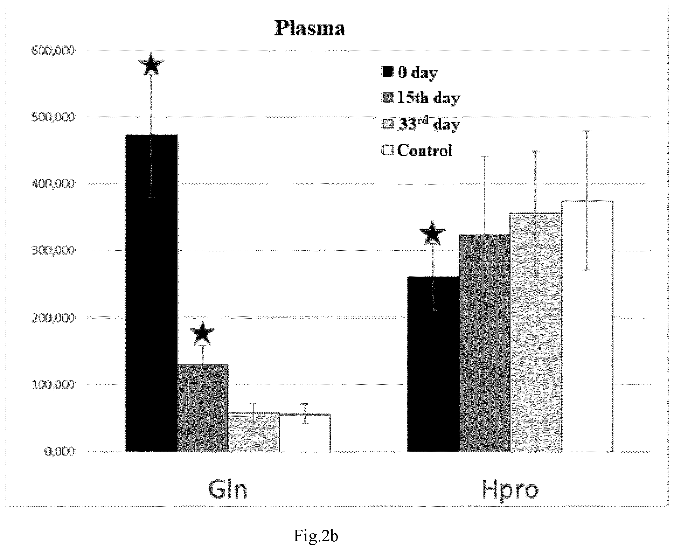

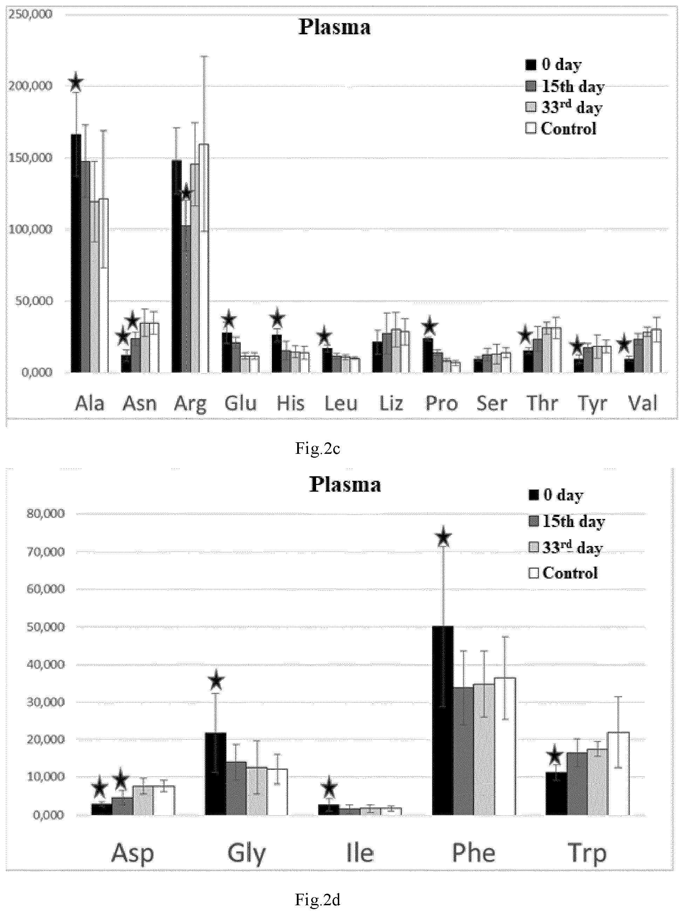

**Fig. 2** - presents average values of amino acids concentration in the blood plasma determined using LC-MS method; n = 40 subjects (children) a) all of the measured amino acids, b) Gln, and Hpro c)amino acids: Ala, Asn, Arg, Glu, His, Leu, Liz, Pro, Ser, Thr, Tyr, Val, excluding Gln, and Hpro d) remaining amino acids excluding the ones mentioned in point b and c. Standard deviation is visually represented in Figures a-d, and statistically significant differences between average concentration of a given amino acid in the group of subjects suffering from ALL leukaemia and the control group are marked with * and represented in Figures b-d.

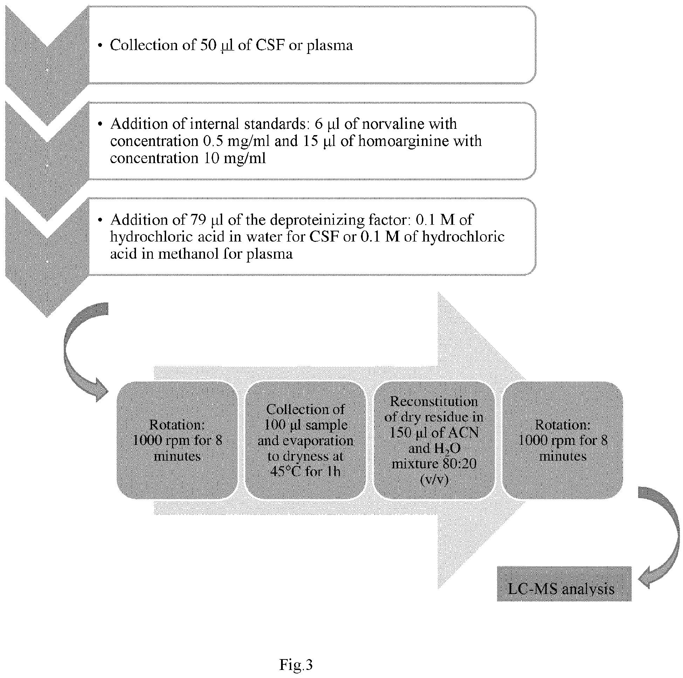

**Fig. 3** - presents the scheme of the new procedure of cerebrospinal fluid, and blood plasma samples preparation for the LC-MS analysis.

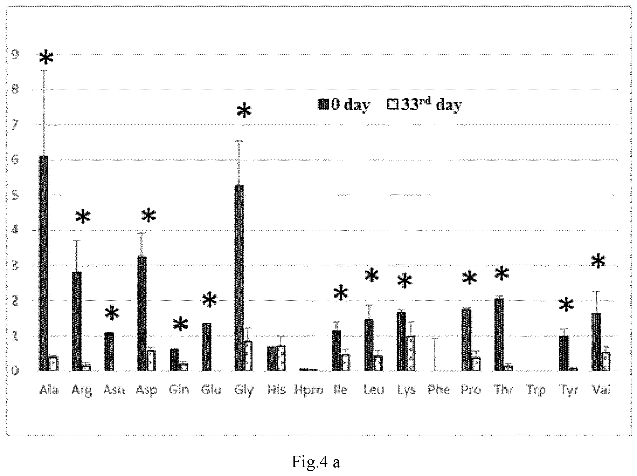

**Fig. 4a** - presents average values of amino acids concentration in the exhaled breath condensates determined using LC-MS/MS method; n = 6 subjects (children) for all of the measured amino acids in the group of children suffering for leukaemia ALL in the moment of diagnosis (before treatment, n=6), and in the group of subjects with disease remission (33 day of treatment, n = 6). Statistically significant differences between average concentration of a given amino acid in the group of subjects suffering from leukaemia ALL at the time of diagnosis (before treatment) and the group with disease remission (33 day of treatment) are marked with * in Figure 4a.

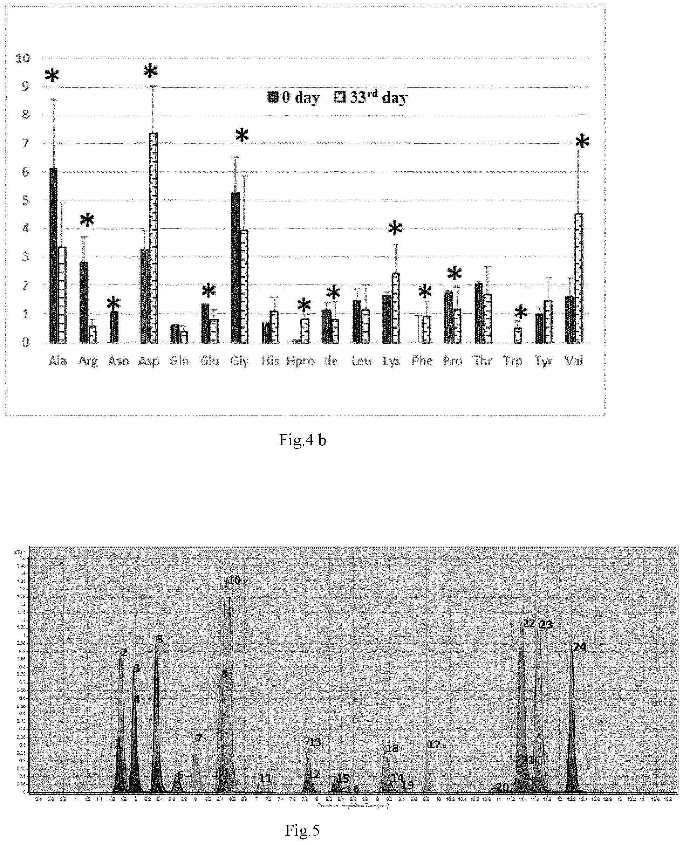

**Fig. 4b** - presents average values of amino acids concentration in the exhaled breath condensates determined using LC-MS/MS method; n = 6 subjects (children) for all of the measured amino acids in the group of children suffering for leukaemia ALL at the time of diagnosis (before treatment, n=6), and in the group of healthy subjects (n = 10). Statistically significant differences between average concentration of a given amino acid in the group of subjects suffering from leukaemia ALL at the time of diagnosis (before treatment, day 0) and the group of healthy subjects (n = 10) are marked with * in Figure 4b.
**Fig. 5** - presents the chromatogram of standards for the analysed amino acids determined using LC-MS/MS method.

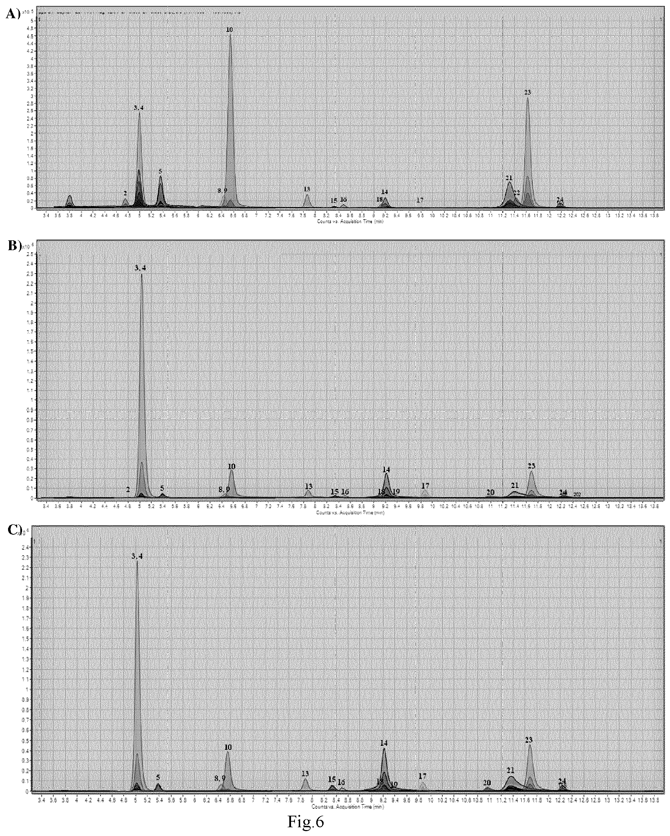

**Fig. 6** - presents the chromatograms of 23 analysed amino acids, and the internal standard (Leucine-d3) in the exhaled breath condensates collected by using CL-MS/MS technique in positive ions mode: from a child with diagnosed leukaemia (A), from a child with disease remission (B), and from a healthy child (C), where: 1 - Tryptophan, 2 - Phenylalanine, 3 - Leucine-D3, 4 - Leucine, 5 - Isoleucine, 6 - Methionine, 7 - Norvaline, 8 - Tyrosine, 9 - Valine, 10 - Proline, 11 - Cysteine, 12 - Hydroxyproline, 13 - Alanine, 14 - Serine, 15 - Threonine, 16 - Glycine, 17 - Glutamine Acid, 18 - Glutamine, 19 - Asparagine, 20 - Aspartic Acid, 21 - Histidine, 22 - Homoarginine, 23 - Arginine, 24 - Lysine.

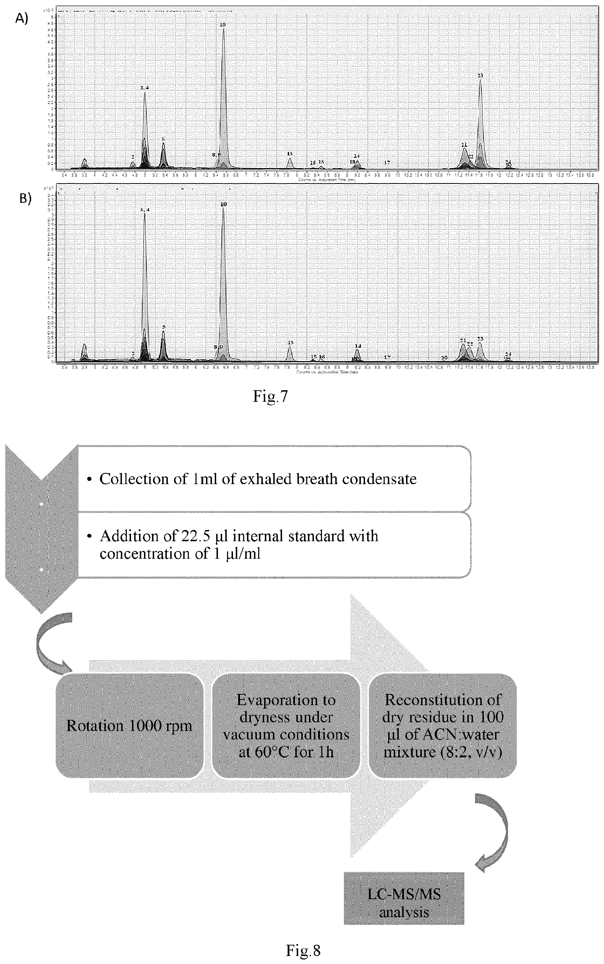

**Fig. 7** - presents the chromatograms of the 23 analysed amino acids, and the internal standard (Leucine-d3) in the exhaled breath condensates collected by using CL-MS/MS technique in positive ions mode: from a patient with type I diabetes (A), from a healthy child (B), where: 1 - Tryptophan, 2 - Phenylalanine, 3 - Leucine-D3, 4 - Leucine, 5 - Isoleucine, 6 - Methionine, 7 - Norvaline, 8 - Tyrosine, 9 - Valine, 10 - Proline, 11 - Cysteine, 12 - Hydroxyproline, 13 - Alanine, 14 - Serine, 15 - Threonine, 16 - Glycine, 17 - Glutamine Acid, 18 - Glutamine, 19 - Asparagine, 20 - Aspartic Acid, 21 - Histidine, 22 - Homoarginine, 23 - Arginine, 24 - Lysine.
**Fig. 8** - presents the scheme of the new procedure of exhaled breath samples preparation for the LC-MS/MS analysis.

The following examples of performance illustrate the invention, while, not restricting it.

### Example 1 (invention)

### Analysis of L-amino acids level in cerebrospinal fluid and human blood plasma samples in the in the group of diseased children and in the control group

### Analysis conditions

The analysis of amino acids was carried out using liquid chromatography coupled with mass spectrometry (LC-MS) with the ChemStation data acquisition system (Agilent Technologies, Santa Clara, CA, USA). The HPLC system was equipped with a pump, an autosampler, a UV detector, and a degasser. Chromatographic separation of amino acids was done on the basis of hydrophilic interaction liquid chromatography HILIC using the Xbridge Amide column with dimension of 3x100 mm, 3,5 µm (Waters) packed with polar sorbent which was placed in a thermostat at the temperature of 25°C. Mobile phase consisted of the phase A: 10 mM ammonium formate (pH3) in water, and B: 10 mM ammonium formate in acetonitrile under the conditions of gradient elution. Flow rate of the mobile phase was 1ml/minute. The working conditions of the mass detector were as follows: temperature of the drying gas (nitrogen): 250°C, flow rate of the drying gas (nitrogen): 12 L/minute, capillary voltage: 3kV, fragmentation voltage dependent on the substance (range from 75 to 125V). The total time of a single analysis of 21 amino acids including column equilibration was 30 minutes. The separation of amino acids was carried out under the conditions of gradient elution.

### Preparation of biological samples for the LC-MS analysis

The choice of the deproteinizing agent resulted from the difference in the total number of proteins between both samples. The number of proteins is much larger in blood plasma samples. Hydrochloric acid serves both as the deproteinization element, and as the solvent. This is why the preparation stage was based on the single-stage liquid-liquid extraction.

As a result of using 0,1 M of HCl in water as a deproteinizing agent was used for cerebrospinal fluid (CSF), the roughage proteins precipitated, and the amino acids of interest remained in the aqueous layer for cerebrospinal fluid where the following stage was to evaporate the sample until it is dry and then dissolve it in the mixture of acetonitrile and H₂O (8:2, v/v).

In the case of blood plasma, 0,1 M of hydrochloric acid in methanol as a deproteinizing agent for blood plasma was used, where after proteins' precipitation the organic layer was moved to new samples, and was evaporated under vacuum until it was dry similarly to CSF (cerebrospinal fluid) samples. This new procedure of sample preparation allowed successful extraction of examined amino acids. Detailed process of the applied procedure is presented in Fig. 3.

### Examined subjects

The subjects of examination were paediatric patients (in the number of 40) from the Department of Pediatrics, Hematology and Oncology (Medical University of Gdańsk, Poland), diagnosed with B-cell acute lymphoblastic leukaemia (BCP-ALL). L-asparaginase is one of the medications used in treatment of these patients in accordance with the 1A protocol. It influences amino acid management in various body fluids (cerebrospinal fluid, blood, urine) before, and during treatment. The levels of certain L-amino acids indicate statistically significant differences in particular stages of therapy when compared with the control group. An attempt to demonstrate the correlation between the level of chosen amino acids at the moment of diagnosis and during chemotherapy was made.

### Results

**Table 1** - presents average values of amino acids' densities in cerebrospinal fluid of children with diagnosed acute lymphoblastic leukaemia (ALL) (n = 40 people / children /) and in the clinical control group (n = 6), marked by the LC-MS method, 0th day means the moment of diagnosis of the disease before starting treatment, 15th day means a stage after chemotherapy treatment, 33rd day means remission of the disease.

**Table 1**

| | | 0 day | | 15th day | | 33rd day | | Control | |
|---|---|---|---|---|---|---|---|---|---|
| | AA | µg/ml | ± SD | µg/ml | ± SD | µg/ml | ± SD | µg/ml | ± SD |
| 1 | Ala | 19.120 | 16.331 | 9.020 | 4.917 | 15.960 | 11.821 | 15.682 | 9.093 |
| 2 | Arg | 16.290 | 4.963 | 10.391 | 5.232 | 21.886 | 11.746 | 31.950 | 8.881 |
| 3 | Asn | 1.141 | 0.385 | 2.350 | 0.401 | 3.420 | 0.819 | 3.440 | 1.000 |
| 4 | Asp | 0.901 | 0.322 | 0.578 | 0.225 | 1.192 | 0.322 | 1.710 | 0.208 |
| 5 | Gln | 603.700 | 139.626 | 270.273 | 62.170 | 201.489 | 46.825 | 87.499 | 28.275 |
| 6 | Glu | 4.241 | 2.069 | 5.480 | 1.878 | 6.184 | 3.220 | 6.911 | 1.794 |
| 7 | Gly | 0.270 | 0.172 | 0.196 | 0.157 | 0.178 | 0.099 | 0.170 | 0.074 |
| 8 | His | 13.870 | 3.417 | 24.374 | 10.155 | 28.766 | 8.284 | 29.800 | 8.049 |
| 9 | Hpro | 387.060 | 73.962 | 103.710 | 37.626 | 64.110 | 22.943 | 56.240 | 14.385 |
| 10 | Ile | 0.230 | 0.144 | 0.160 | 0.199 | 0.150 | 0.106 | 0.130 | 0.063 |
| 11 | Leu | 1.800 | 0.189 | 1.500 | 0.197 | 1.400 | 0.217 | 1.300 | 0.181 |
| 12 | Lys | 12.342 | 5.761 | 13.463 | 7.246 | 15.084 | 6.131 | 15.872 | 10.993 |
| 13 | Phe | 0.510 | 0.238 | 0.310 | 0.119 | 0.270 | 0.094 | 0.267 | 0.123 |
| 14 | Pro | 10.021 | 0.930 | 3.845 | 0.501 | 3.027 | 0.411 | 2.987 | 0.176 |
| 15 | Ser | 4.210 | 0.667 | 3.021 | 1.098 | 2.337 | 1.274 | 2.670 | 0.828 |
| 16 | Thr | 7.091 | 0.560 | 4.211 | 1.543 | 3.061 | 0.420 | 13.570 | 0.880 |
| 17 | Trp | 0.090 | 0.015 | 0.070 | 0.013 | 0.060 | 0.010 | 0.050 | 0.013 |
| 18 | Tyr | 2.050 | 0.617 | 3.540 | 0.623 | 3.851 | 1.934 | 4.110 | 1.101 |
| 19 | Val | 4.650 | 0.693 | 6.765 | 1.149 | 7.024 | 0.808 | 14.498 | 3.958 |

**Table 2** - presents average values of amino acids' densities in blood plasma of children with diagnosed acute lymphoblastic leukaemia (ALL) (n = 40 people / children) and in the clinical control group (n = 6), marked by the LC-MS method, 0 day means the moment of diagnosis of the disease before starting treatment, 15th day means a stage after chemotherapy treatment, 33rd day means remission of the disease.

**Table 2**

| | | 0th day | | 15th day | | 33rd day | | Control | |
|---|---|---|---|---|---|---|---|---|---|
| | AA | µg/ml | ± SD | µg/ml | ± SD | µg/ml | ± SD | µg/ml | ± SD |
| 1 | Ala | 166,130 | 29,296 | 147,531 | 25,198 | 119,071 | 28,363 | 121,072 | 47,791 |
| 2 | Arg | 147,680 | 22,881 | 102,490 | 17,827 | 145,310 | 29,048 | 159,510 | 61,213 |
| 3 | Asn | 11,658 | 4,181 | 23,504 | 4,713 | 34,529 | 9,692 | 34,462 | 8,050 |
| 4 | Asp | 3,020 | 0,586 | 4,697 | 1,831 | 7,590 | 2,049 | 7,680 | 1,507 |
| 5 | Gln | 472,205 | 92,365 | 129,030 | 29,228 | 57,418 | 13,446 | 56,008 | 14,338 |
| 6 | Glu | 27,480 | 7,412 | 20,684 | 4,108 | 11,378 | 2,268 | 11,378 | 2,268 |
| 7 | Gly | 21,764 | 10,647 | 13,927 | 4,703 | 12,585 | 6,999 | 12,179 | 3,967 |
| 8 | His | 26,160 | 4,531 | 15,234 | 6,347 | 14,283 | 4,139 | 13,646 | 4,505 |
| 9 | Hpro | 261,320 | 49,592 | 323,575 | 117,393 | 356,442 | 91,770 | 375,144 | 104,240 |
| 10 | Ile | 2,850 | 1,679 | 1,595 | 1,189 | 1,788 | 1,078 | 1,764 | 0,591 |
| 11 | Leu | 16,529 | 2,453 | 11,084 | 2,054 | 10,497 | 1,882 | 9,889 | 0,912 |
| 12 | Lys | 20,871 | 8,447 | 26,926 | 14,493 | 29,866 | 12,140 | 28,296 | 9,070 |
| 13 | Phe | 50,111 | 21,324 | 33,773 | 9,854 | 34,770 | 8,863 | 36,423 | 10,920 |
| 14 | Pro | 23,328 | 1,237 | 13,550 | 1,870 | 8,359 | 1,089 | 6,659 | 1,728 |
| 15 | Ser | 9,317 | 1,427 | 12,083 | 4,391 | 12,614 | 6,879 | 13,712 | 3,369 |
| 16 | Thr | 14,975 | 2,010 | 23,285 | 8,475 | 30,855 | 4,235 | 30,968 | 7,350 |
| 17 | Trp | 11,216 | 2,113 | 16,467 | 3,779 | 17,441 | 2,006 | 21,940 | 9,422 |
| 18 | Tyr | 9,259 | 2,786 | 16,993 | 3,076 | 17,985 | 7,977 | 18,202 | 4,359 |
| 19 | Val | 9,350 | 1,621 | 23,000 | 3,906 | 28,094 | 3,234 | 29,811 | 8,599 |

### Example 2 (invention)

### Analysis of L-amino acids level in exhaled breath condensate samples in the in the group of diseased children and in the control group

### Conditions of the analysis

Analysis of amino acid in exhaled breath condensate samples was performed by liquid chromatography coupled with tandem mass spectrometry (LC-MS/MS) with MassHunter data acquisition system (Agilent Technologies, Santa Clara, CA, USA). The HPLC system was equipped with a pump, autosampler, UV detector, and degasser. Chromatographic separation of amino acids was conducted on the basis of hydrophilic interaction chromatography (HILIC) using a XBridge Amide column, size 3 x 100 mm, 3.5 µm (Waters), which was placed in a thermostat at 25°C temperature. The mobile phase consisted of phase A: 10 mM ammonium formate (pH3) in water, and B: 10 mM ammonium formate in acetonitrile under gradient elution conditions. The flow of mobile phase was 0.8 mL/min. Column temperature was 25°C. The working conditions of the mass detector were as follows: temperature of the drying gas (nitrogen): 350°C, flow of the drying gas (nitrogen): 8 L/min, temperature of the sheath gas (nitrogen): 350°C, flow of the sheath gas (nitrogen): 11 L/min, collision energy depending on the monitored daughter ion (range from 5 to 70). The duration of a single analysis of 21 amino acids together with column equilibration is 25 minutes. Separation of amino acids occurred under gradient elution conditions. Under selected, optimized and above-described conditions, a chromatogram of the standards of the analysed amino acids was obtained, as illustrated in Fig. 2.

### Preparation of biological samples for the LC-MS/MS analysis

A fast, simple and solvent-free procedure for sample preparation for chromatographic analysis was developed, which is based on condensation of a sample of exhaled breath condensate by its evaporation under vacuum conditions (exhaled breath condensate). Due to the very low levels of amino acid concentrations in exhaled breath condensates, a new procedure for sample preparation has been developed before further analysis, using liquid chromatography technique coupled with tandem mass spectrometry (LC-MS/MS), which involves evaporation of the sample under vacuum conditions using concentrator CentriVap (LabConco, (batch number 121168609). The stages consist of adding an internal standard: labelled leucine, Leu-d3, to the sample of exhaled breath condensate of a volume of 1mL, rotation: 1000 rpm/8 minutes. The sample is then taken and evaporated to dryness at 60°C temperature during1h. The next step involves dissolving the dry residue in 100 µL of a water-acetonitrile mixture (8: 2, v/v) and again rotating 10000 rpm/8 minutes followed by LC-MS/MS analysis. The detailed course of the aforesaid procedure is presented in Fig. 4.

This choice of sample preparation procedure resulted from the fact that the exhaled breath condensate is virtually free from proteins and does not require the use of a deproteinizing reagent. The new method of sample preparation for analysis allowed concentration of the sample, which translated into increase of sensitivity of the method, and then enabled effective further analysis of the tested amino acids with the use of LC-MS/MS technique (Fig. 4.)

### Examined subjects

6 paediatric patients of the Department of Paediatrics, Haematology and Oncology in Gdańsk, with diagnosed B-cell precursor acute lymphoblastic leukaemia (BCP-ALL) were subjects to the study. One of the drugs used to treat these patients according to protocol 1A is L-asparaginase, which affects the amino acid balance in human body. We present the results of tests based on comparing the concentrations of amino acids in exhaled breath condensates before and during the treatment (Table 1). Levels of some L-amino acids show statistically significant differences at individual stages of the therapy (Fig. 1a illustrates the moment of diagnosis in relation to the remission stage of the disease) and Fig. 1b presents the moment of diagnosis compared with the clinical control group. An attempt was made to show a correlation between the level of selected amino acids at the time of diagnosis and during chemotherapy.

### Results

Table 1 shows the average concentration values determined using the developed LC-MS/MS method at the time of diagnosis (0^{th} day, n = 6), during remission of the disease (33^{rd} day, n = 6) and in the group of healthy children (n = 10).

**Table 3** - presents average values of amino acid concentrations in exhaled breath condensates of children with diagnosed acute lymphoblastic leukaemia ALL (n = 6 subjects/children) at the time of diagnosis, during remission of the disease and in the clinical control group (n = 10), determined by LC-MS / MS method, 0 day means the moment of diagnosis of the disease before starting the treatment, 33^{rd} day means remission of the disease.

**Table 3**

| | | 0 day | | 33rd day (remission) | | Control | |
|---|---|---|---|---|---|---|---|
| | AA | ng/mL | ± SD | ng/mL | ± SD | ng/mL | ± SD |
| 1 | Ala | 6.10 | 2.4352 | 0.39 | 0.0418 | 3.34 | 1.5587 |
| 2 | Arg | 2.80 | 0.9123 | 0.15 | 0.0896 | 0.55 | 0.2417 |
| 3 | Asn | 1.06 | 0.0345 | 0.00 | 0.00 | 1.00 | 0.4751 |
| 4 | Asp | 3.23 | 0.6843 | 0.57 | 0.1068 | 7.34 | 1.6776 |
| 5 | Gln | 0.62 | 0.0284 | 0.18 | 0.0745 | 0.58 | 0.1927 |
| 6 | Glu | 1.33 | 0.0162 | 0.00 | 0.00 | 0.78 | 0.3738 |
| 7 | Gly | 5.25 | 1.2863 | 0.83 | 0.4007 | 3.93 | 1.9350 |
| 8 | His | 0.70 | 0.0056 | 0.72 | 0.2906 | 1.09 | 0.5007 |
| 9 | Hpro | 0.07 | 0.0098 | 0.03 | 0.0185 | 0.81 | 0.1668 |
| 10 | Ile | 1.14 | 0.2486 | 0.44 | 0.1750 | 0.78 | 0.6228 |
| 11 | Leu | 1.45 | 0.4282 | 0.41 | 0.1646 | 1.13 | 0.8890 |
| 12 | Lys | 1.64 | 0.1028 | 0.99 | 0.3922 | 2.43 | 1.0180 |
| 13 | Phe | 0.00 | 0.00 | 0.00 | 0.00 | 0.89 | 0.5255 |
| 14 | Pro | 1.74 | 0.0583 | 0.37 | 0.1925 | 1.17 | 0.7892 |
| 15 | Ser | 6.15 | 0.667 | 1.99 | 0.7152 | 173.81 | 36.0134 |
| 16 | Thr | 2.04 | 0.0968 | 0.15 | 0.0810 | 1.69 | 0.9707 |
| 17 | Trp | 0.00 | 0.00 | 0.00 | 0.00 | 0.50 | 0.2355 |
| 18 | Tyr | 1.00 | 0.2096 | 0.06 | 0.0364 | 1.44 | 0.8382 |
| 19 | Val | 1.62 | 0.6425 | 0.51 | 0.1958 | 4.51 | 2.2524 |

Graphically represented levels of labelled amino acids in the groups of subjects: in the group of patients at the time of diagnosis of leukaemia (0 day, n = 6) and during disease remission (33rd day, n = 6) they are presented in the form of a bar graph in Fig. 1a. Fig. 1b, on the other hand, shows average values of amino acid concentrations in exhaled breath condensates of patients before the treatment (diagnosis, 0 day) and of clinical control group of healthy control paediatric group (n = 10).

Obtained records of the test apparatus in the form of chromatographic peaks show the chromatograms obtained with the use of LC-MS/MS method: including for the analysed benchmark substances (Fig. 5) and for 23 amino acids in exhaled breath samples collected from a child diagnosed with leukaemia (Fig. 6A), from a child during remission of the disease (Fig. 6B) and from a healthy child (Fig. 6C).

### Example 3 (invention)

### Analysis of L-amino acids level in exhaled breath condensate samples in the in the group of diseased children and in the control group

### The basis and purpose of testing of the group of children with type 1 diabetes

The test evaluated the exhaled breath condensation taken at specific time points:

### Type 1 diabetes:

1. Evaluation after a stabilized one-year treatment.
2. Evaluation during control visits.
3. Evaluation of the occurrence of acute or chronic complications and the stage of causative treatment.
4. Before and after the possible change of treatment.

**Table 4 - presents average values of amino acid concentrations in exhaled breath condensates in children with type 1 diabetes (n = 19 subjects/children) and in the clinical control group (n = 54), determined by the LC-MS/MS method.**

| | Control group (n=54) | | Diabetology (n=19) | |
|---|---|---|---|---|
| AA | Average [ng/mL] | ±SD | Average [ng/mL] | ±SD |
| Tryptophane | 0.50 | 0.2355 | 0.55 | 0.2285 |
| Phenylalanine | 0.89 | 0.5255 | 0.69 | 0.3471 |
| Leucine | 1.33 | 0.8890 | 2.98 | 0.9862 |
| Isoleucine | 0.78 | 0.6228 | 1.68 | 0.7513 |
| Methionine | 1.37 | 0.2931 | 0.11 | 0.0030 |
| Norvaline | 0.49 | 0.3055 | 1.11 | 0.4297 |
| Valine | 4.51 | 2.2524 | 3.67 | 1.1677 |
| Tyrosine | 1.44 | 0.8382 | 2.46 | 1.0591 |
| Proline | 1.17 | 0.7892 | 2.49 | 0.8779 |
| Alanine | 3.34 | 1.5587 | 7.21 | 3.4095 |
| Hydroxyproline | 0.81 | 0.1668 | 0.80 | 0.0941 |
| Threonine | 1.99 | 0.9707 | 3.90 | 1.5489 |
| Glycine | 3.93 | 1.9350 | 11.57 | 5.1694 |
| Glutamine | 0.58 | 0.1927 | 0.62 | 0.1864 |
| Serine | 17.,81 | 76.0134 | 70.65 | 34.6106 |
| Asparagine | 1.00 | 0.4751 | 1.16 | 0.5726 |
| Glutamic acid | 0.98 | 0.3738 | 1.59 | 0.7781 |
| Aspartic acid | 7.34 | 3.6776 | 7.27 | 3.3787 |
| Homoarginine | 0.51 | 0.1608 | 0.60 | 0.2082 |
| Histidine | 1.09 | 0.5007 | 4.10 | 2.0094 |
| Arginine | 0.55 | 0.2417 | 1.32 | 0.6609 |
| Lysine | 2.43 | 1.2180 | 3.45 | 1.71830 |

Obtained records of the test apparatus in the form of chromatographic peaks show the chromatograms obtained by the LC-MS/MS technique: including for the analysed benchmark substances (Figure 5) and for 23 amino acids in exhaled breath samples from a child with type 1 diabetes (Fig 7A) and a healthy child (Figure 7B).

### Description of the protocol 1A (not part of an invention)

Initial diagnosis of acute leukaemia is based in the first place on classic criteria, i.e. interview, physical symptoms and laboratory blood tests. It should be noted that these symptoms are often unclear and non-specific and due to that they can be mistaken with the symptoms of common childhood illnesses which may delay the proper diagnosis.

In the next stage, specialized cytochemical, morphological, cytogenetic tests and immunophenotypification of the collected bone marrow are performed. After diagnosing leukaemia on the basis of the performed clinical tests, and determining its type, the clinician chooses the patient's treatment protocol. The acute lymphoblastic leukaemia treatment protocol 1A (PROTOCOL 1A) is the first protocol that covers the expected treatment time of 33 days. On the same day as the diagnosis is made (after performing the myelogram of bone marrow and lumbar puncture for the assessment of cerebrospinal fluid), the treatment begins. At the beginning, the patient is given a dose of steroids adapted to his/her age and weight, a child receives Encorton for one week, then on the eighth day, the response to steroid therapy is checked. If the response is good, the child remains in the intermediate risk group (the results are visible in the blood smear). On the twelfth day, a lumbar puncture is performed during which methotrexat is administered to the spinal cord. Another important day is the 15th day, in which the myelogram of bone marrow is performed to check if the remission of the disease has been achieved (i.e. a decrease in the number of tumour cells, called blasts, in bone marrow to less than 5% and the withdrawal of clinical symptoms associated with the disease). The therapy ends on the 33rd day, when the lumbar puncture with methotrexate and myelogram are performed. A child should be in complete remission.

### References:

Krawczuk-Rybak M. *Kompendium onkologii dzieci* 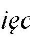 *cej dla studentów VI roku wydzia* 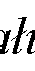 *u lekarskiego AM.* Uniwersytet Medyczny Bialystok, Bialystok 2004.
Qureshi A., Hall G. Leukaemias: a review. Paediatrics and Child Health, 2013; 23:461-466.
Szczeklik A. *Interna Szczeklika. Podr* 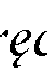 *cznik chorób wewn* 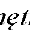 *trznych,* Medycyna Praktyczna, Kraków 2013.
Pui C., Robison L., Look A. Acute lymphoblastic leukemia. Lancet, 2008; 371:1030-1043.
Zipursky A., Poon A., Doyle J. Leukemia in Down syndrome: A review. Journal of Pediatric Hematology and Oncology, 1992; 9:139-49.
Colby-Graham M.F., Chordas C. The childhood leukemias. Journal of Pediatric Nursing, 2003; 18:87-95.
Arya L.S. Acute lymphoblastic leukemia: current treatment. Apollo Medicine, 2005; 2:319-323.
Peng C.T., Wu K.H., Lan S.J., Tsai J.J., Tsai F.J., Tsai C.H.. Amino acid concentrations in cerebrospinal fluid in children with acute lymphoblastic leukemia undergoing chemotherapy. European Journal of Cancer, 2005; 41:1158-1163.

## Claims

1. A method for monitoring of amino acids in a biological material, comprising the steps of:
a) preparation of a sample of a biological material which is an exhaled breath condensate, wherein 1 mL of an internal standard is added to the sample, the internal standard is norvaline and homoarginine, labelled deuterated leucine Leu-d3,
b) centrifugation of a sample at 1000 rpm / 8 minutes,
c) subsequent evaporation to dryness at 45-60°C during 1h,
d) dissolving of a dry residue in 100 µL of a mixture of acetonitrile and H₂O in a ratio of 8:2 (v/v) and subsequent centrifugation at 10000 rpm /8 minutes, and
e) chromatographic analysis with a mass detector by liquid chromatography coupled with tandem mass spectrometry (LC-MS/MS) performed under conditions wherein:
- 21 amino acids are separated by hydrophilic interaction chromatography (HILIC), with the use of a column packed with polar sorbent,
- a mobile phase consists of phase A): 10 mM ammonium formate (pH 3) in water and B): 10 mM ammonium formate in acetonitrile under gradient elution conditions,
- the mobile phase flow is 0.8 - 1 mL/min,
- the column temperature is 25°C,
- a drying gas temperature:250-350°C, flow of the drying gas: 8-12 L/min, the drying gas temperature is: 350°C, flow of the drying gas: 11 L/min, collision energy depending on the monitored production (range from 5 to 70),
- the duration of a single analysis of 21 amino acids with column equilibration is 25 - 30 min, and amino acid separation occurred under gradient elution conditions;

2. A method for monitoring of amino acids in a biological material, comprising the following steps of:
a) preparation of a sample of one biological material which is a cerebrospinal fluid or a blood plasma
b) addition of a deproteinizing agent which is a 0.1 M solution of hydrochloric acid in water for cerebrospinal fluid or 0.1 M hydrochloric acid in methanol for blood plasma,
c) centrifugation of a sample at 1000 rpm / 8 minutes,
d) subsequent evaporation to dryness at 45-60°C during 1h,
e) dissolving of a dry residue in 100 µL of a mixture of acetonitrile and H₂O in a ratio of 8:2 (v/v) and subsequent centrifugation: 10000 rpm /8 minutes, and
f) chromatographic analysis with a mass detector by liquid chromatography coupled with tandem mass spectrometry (LC-MS/MS), wherein:
- 21 amino acids are separated by hydrophilic interaction chromatography (HILIC), with the use of a column packed with a polar sorbent,
- a mobile phase consists of phase A): 10 mM ammonium formate (pH 3) in water and B): 10 mM ammonium formate in acetonitrile under gradient elution conditions,
- the mobile phase flow is 0.8 - 1 mL/min,
- the column temperature is 25ºC,
- drying gas temperature: 250-350°C, flow of the drying gas: 8-12 L/min, drying gas temperature is: 350°C, flow of the drying gas: 11 L/min, collision energy depending on the monitored production (range from 5 to 70),
- the duration of a single analysis of 21 amino acids with column equilibration is 25 - 30 min, - amino acid separation occurred under gradient elution conditions.

3. The use of the method as defined in claim 1 for monitoring of the level of amino acids in patients with lymphoblastic leukaemia and/or type 1 diabetes.

4. The use according to claim 3 wherein the monitoring occurs at the time of diagnosis and during chemotherapy.

5. The use according to claim 3 wherein patients belong to the paediatric population.

## Patentansprüche

1. Verfahren zum Überwachen von Aminosäuren in einem biologischen Material, umfassend die Schritte:
a) Vorbereitung einer Probe eines biologischen Materials, das ein ausgeatmetes Atemkondensat ist, wobei 1 mL eines internen Standards zu der Probe hinzugefügt wird, wobei der interne Standard Norvalin und Homoarginin ist, das mit deuteriertem Leucin Leu-d3 markiert ist,
b) Zentrifugation einer Probe bei 1000 U/min / 8 Minuten,
c) anschließendes Eindampfen zur Trockne bei 45-60°C während 1h,
d) Auflösen eines trockenen Rückstands in 100 µL einer Mischung aus Acetonitril und H₂O im Verhältnis 8:2 (v/v) und anschließende Zentrifugation bei 10000 U/min /8 Minuten, und
e) chromatographische Analyse mit einem Massendetektor durch Flüssigchromatographie mit Tandem-Massenspektrometrie-Kopplung (LC-MS/MS) unter Bedingungen, wobei:
- 21 Aminosäuren durch hydrophile Interaktionschromatographie (HILIC) getrennt werden, unter Verwendung einer mit polarem Sorptionsmittel gefüllten Säule,
- eine mobile Phase aus Phase A): 10 mM Ammoniumformiat (pH 3) in Wasser und B): 10 mM Ammoniumformiat in Acetonitril besteht, unter Gradientenelutionsbedingungen,
- der Durchfluss der mobilen Phase 0,8 - 1 mL/min beträgt,
- die Säulentemperatur 25°C beträgt,
- die Temperatur eines Trocknungsgases: 250-350°C, Durchfluss des Trocknungsgases: 8 - 12 L/min, die Temperatur des Trocknungsgases: 350°C beträgt, Durchfluss des Trocknungsgases: 11 L/min, Kollisionsenergie in Abhängigkeit von der überwachten Produktion (Bereich von 5 bis 70),
- die Dauer einer einzelnen Analyse von 21 Aminosäuren mit Säulenäquilibrierung 25 - 30 Minuten beträgt, und die Aminosäurentrennung unter Gradientenelutionsbedingungen erfolgte;

2. Verfahren zum Überwachen von Aminosäuren in einem biologischen Material, umfassend die folgenden Schritte:
a) Vorbereitung einer Probe eines biologischen Materials, das eine Zerebrospinalflüssigkeit oder ein Blutplasma ist,
b) Zugabe eines Deproteinierungsmittels, das eine 0,1 M Lösung von Salzsäure in Wasser für Zerebrospinalflüssigkeit oder 0,1 M Salzsäure in Methanol für Blutplasma ist,
c) Zentrifugation einer Probe bei 1000 U/min / 8 Minuten,
d) anschließendes Eindampfen zur Trockne bei 45-60°C während 1h,
e) Auflösen eines trockenen Rückstands in 100 µL einer Mischung aus Acetonitril und H₂O im Verhältnis 8:2 (v/v) und anschließende Zentrifugation: 10000 U/min /8 Minuten, und
f) chromatographische Analyse mit einem Massendetektor durch Flüssigchromatographie mit Tandem-Massenspektrometrie-Kopplung (LC-MS/MS), wobei:
- 21 Aminosäuren durch hydrophile Interaktionschromatographie (HILIC) getrennt werden, unter Verwendung einer mit polarem Sorptionsmittel gefüllten Säule,
- eine mobile Phase aus Phase A): 10 mM Ammoniumformiat (pH 3) in Wasser und B): 10 mM Ammoniumformiat in Acetonitril besteht, unter Gradientenelutionsbedingungen,
- der Durchfluss der mobilen Phase 0,8 - 1 mL/min beträgt,
- die Säulentemperatur 25°C beträgt,
- die Temperatur eines Trocknungsgases: 250-350°C, Durchfluss des Trocknungsgases: 8 - 12 L/min, die Temperatur des Trocknungsgases: 350°C beträgt, Durchfluss des Trocknungsgases: 11 L/min, Kollisionsenergie in Abhängigkeit von der überwachten Produktion (Bereich von 5 bis 70),
- die Dauer einer einzelnen Analyse von 21 Aminosäuren mit Säulenäquilibrierung 25 - 30 Minuten beträgt, die Aminosäurentrennung unter Gradientenelutionsbedingungen erfolgte.

3. Verwendung des Verfahrens nach Anspruch 1 zur Überwachung des Aminosäurespiegels bei Patienten mit lymphoblastischer Leukämie und/oder Typ-1-Diabetes.

4. Verwendung nach Anspruch 3, wobei die Überwachung zum Zeitpunkt der Diagnose und während der Chemotherapie erfolgt.

5. Verwendung nach Anspruch 3, wobei die Patienten zur pädiatrischen Bevölkerungsgruppe gehören.

## Revendications

1. Procédé de surveillance des acides aminés dans une matière biologique comprenant les étapes suivantes :
a) la préparation d'un échantillon d'une matière biologique, laquelle est un condensat d'haleine exhalée, où 1 ml d'un étalon interne est ajouté à l'échantillon, l'étalon interne étant la norvaline et l'homoarginine, marquée leucine deutérée Leu-d3,
b) la centrifugation d'un échantillon à 1 000 tr/m / 8 minutes,
c) l'évaporation subséquente à sec à 45-60 °C pendant 1h,
d) la dissolution d'un résidu sec dans 100 µL d'un mélange d'acétonitrile et de H₂O dans un rapport de 8:2 (v/v) et la centrifugation subséquente à 10 000 tr/min / 8 minutes, et
e) l'analyse chromatographique à l'aide d'un détecteur de masse par chromatographie liquide couplée à la spectrométrie de masse en tandem (LC-MS/MS) réalisée dans des conditions où :
- 21 acides aminés sont séparés par chromatographie d'interaction hydrophile (HILIC), à l'aide d'une colonne remplie de sorbant polaire,
- une phase mobile est constituée de la phase A) : 10 mM de formiate d'ammonium (pH 3) dans l'eau et de la phase B) : 10 mM de formiate d'ammonium dans l'acétonitrile dans des conditions d'élution par gradient,
- le débit de la phase mobile est de 0,8 à 1 mL/min,
- la température de la colonne est de 25 °C,
- une température de gaz de séchage : 250-350 °C, le débit du gaz de séchage : 8 à 12 L/min, la température du gaz de séchage est de : 350 °C, le débit du gaz de séchage : 11 L/min, l'énergie de collision dépendant de la production surveillée (plage comprise entre 5 et 70),
- la durée d'une seule analyse de 21 acides aminés à équilibrage de la colonne est de 25 à 30 min, et la séparation des acides aminés s'est produite dans des conditions d'élution par gradient ;

2. Procédé de surveillance des acides aminés dans une matière biologique comprenant les étapes suivantes :
a) la préparation d'un échantillon d'une matière biologique, laquelle est un liquide céphalo-rachidien ou un plasma sanguin,
b) l'addition d'un agent déprotéinisant, lequel est une solution d'acide chlorhydrique de 0,1 M dans l'eau pour le liquide céphalo-rachidien ou d'acide chlorhydrique de 0,1 M dans le méthanol pour le plasma sanguin,
c) la centrifugation d'un échantillon à 1 000 tr/m / 8 minutes,
d) l'évaporation subséquente à sec à 45-60 °C pendant 1h,
e) la dissolution d'un résidu sec dans 100 µL d'un mélange d'acétonitrile et de H₂O dans un rapport de 8:2 (v/v) et la centrifugation subséquente : 10 000 tr/min / 8 minutes, et
f) l'analyse chromatographique à l'aide d'un détecteur de masse par chromatographie liquide couplée à la spectrométrie de masse en tandem (LC-MS/MS), où :
- 21 acides aminés sont séparés par chromatographie d'interaction hydrophile (HILIC), à l'aide d'une colonne remplie d'un sorbant polaire,
- une phase mobile est constituée de la phase A) : 10 mM de formiate d'ammonium (pH 3) dans l'eau et de la phase B) : 10 mM de formiate d'ammonium dans l'acétonitrile dans des conditions d'élution par gradient,
- le débit de la phase mobile est de 0,8 à 1 mL/min,
- la température de la colonne est de 25 ºC,
- la température du gaz de séchage : 250-350 °C, le débit du gaz de séchage : 8 à 12 L/min, la température du gaz de séchage est de : 350 °C, le débit du gaz de séchage : 11 L/min, l'énergie de collision dépendant de la production surveillée (plage comprise entre 5 et 70),
- la durée d'une seule analyse de 21 acides aminés à équilibrage de la colonne est de 25 à 30 min, - la séparation des acides aminés s'est produite dans des conditions d'élution par gradient.

3. Utilisation de la méthode selon la revendication 1 pour surveiller le taux d'acides aminés chez des patients atteints de leucémie lymphoblastique et/ou de diabète de type 1.

4. Utilisation selon la revendication 3, où la surveillance a lieu au moment du diagnostic et pendant la chimiothérapie.

5. Utilisation selon la revendication 3, où les patients appartiennent à la population pédiatrique.
